(19) Europäisches Patentamt
European Patent Office
Office européen des brevets

(11) Publication number: **0 396 080 B1**

(12) **EUROPEAN PATENT SPECIFICATION**

(45) Date of publication of patent specification: **18.01.95**

(51) Int. Cl.⁶: **C07F 9/09**, A61K 31/66

(21) Application number: **90108222.2**

(22) Date of filing: **30.04.90**

(54) **New serine derivatives, process for their preparation and their use in human therapy.**

(30) Priority: **03.05.89 IT 2035789**

(43) Date of publication of application:
**07.11.90 Bulletin 90/45**

(45) Publication of the grant of the patent:
**18.01.95 Bulletin 95/03**

(84) Designated Contracting States:
**AT BE CH DE DK ES FR GB GR IT LI LU NL SE**

(56) References cited:
**WO-A-87/05024**
**GB-A- 2 057 872**

**BRITISH JOURNAL OF PHARMACOL- OGY, vol. 67, September 1979 E. BIGON et al"Pharmacolo- gical effects of phosphatidyl-serine liposomes: The role of lysophoshatidylserine, pages 611-616**

**BRITISH JOURNAL OF PHARMACOL- OGY, vol. 66, May 1979 E. BIGON at al."Pharmacolo- gical effects of phosphatidyl-serine liposomes: Regulation ofglycolysis and energy levelin brain", pages 167-174**

(73) Proprietor: **FIDIA S.p.A.**
**Via Ponte della Fabbrica 3-A**
**I-35031 Abano Terme (Padova) (IT)**

(72) Inventor: **Della Valle, Francesco**
**Via Cerato, 14**
**I-35100 Padova (IT)**
Inventor: **Romeo, Aurelio**
**Viale Ippocrate, 93**
**I-00161 Rome (IT)**

(74) Representative: **Gervasi, Gemma, Dr. et al**
**NOTARBARTOLO & GERVASI Srl**
**Viale Bianca Maria 33**
**I-20122 Milan (IT)**

**EP 0 396 080 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Field of the Invention

The present invention refers to new serine derivatives of general formula (I)

```
           A     O        NH3(+)
           |     ||       |
     (I)   CH-O-P-O-CH2-CH-COO(-) • X(+)
           |     |
           B     OH
```

in which A = H, $-(CH_2)n$ - O-CO-$R_1$, n being 1 or 2,
and B = $-(CH_2)_m$-O-CO-$R_2$, m being 1, 2, 3 or 4, with the limitation that when A = $-(CH_2)_n$ O-CO-$R_1$, m = 1, or 2 and wherein $R_1$ and $R_2$, equal or different, are radicals of acids of the aliphatic, aromatic, araliphatic, alicyclic or heterocyclic series, $R_1$ and $R_2$ being preferably radicals of acids of the aliphatic series with a maximum of 20 carbon athoms, and which may be saturated, mono- or polyunsaturated, such as for instance formic, acetic, propionic, butyric, valerianic, capronic, caprinic, lauric, myristic, palmitic, palmitoleic, stearic, oleic, linoleic, linolenic, arachidonic acid; they may also represent radicals of hydroxy acids, such as lactic acid, of aminoacids such as glycine, or of bicarboxylic acids, such as succinic, glutaric, malonic or maleic acid.

When $R_1$ and $R_2$ are aromatic acid radicals, the preferred acids are the ones with only one benzene ring, in particular benzoic acid and its derivatives having as ring substituents methyl, hydroxy, amino or carboxyl groups, as for instance p-aminobenzoic acid, salicylic acid or phthalic acid.

X may be an alkali or earth-alkali metal, a primary, secundary, tertiary amine or a quaternary ammonium, aliphatic, aromatic or heterocyclic base.

X is preferably potassium, sodium, ammonium, calcium, magnesium, piperazine, proglycamine (3-amino-1,2-dihydroxy propane), lysine, meglumine (N-methyl glucamine), betaine, choline, aminobutanol, ethanolamine, arginine, carnitine, diethanolamine, dimethylamino ethanol, dimethyl piperazine.

The present invention refers furthermore to a process for preparing the new serine derivatives, and to their use in the therapy of human pathologies associated with a slow-down of cerebral metabolism, or as hypolipidemizing agents.

Prior art

Phosphatidyl-serine (PS) and lysophosphatylserine (Lyso PS), serine phospholipid derivatives, are natural derivatives of known pharmacological activity. In particular, it is known that the natural phospholipid derivatives have the property of raising the glycemic and histamine levels, producing a glucose accumulation in cerebral tissues, when administered endovenously to rodents (Bigon E., Boarato E., Bruni A., Leon A., Toffano G.,: Pharmacological effects of phosphatidylserine liposomes: regulation of glycolysis and energy level in brain. Br. J. Pharmac. 66, 167-174 (1979) and Pharmacological effects of phosphatidylserine liposomes: the role of lysophosphatidylserine. Br. J. Pharmac. 67, 611-616 (1979); Bruni A., Bigon E., Battiatella A., Boarato E., Mietto L., Toffano G.: Lysophosphatidylserine as histamine releaser in mice and rats. Agents & Actions, 14, 619-625 (1984).

It was observed, however (Bruni A., Bigon E., Battiatella A., Boarato E, Mietto l., Toffano G.: Lysophosphatidylserine as histamine releaser in mice and rats. Agent & Action. 14, 619-625 (1984)) that successive administrations of said products lead to a rapid desensibilization and hence to a lower pharmacological response.

It was also observed (Boarato E., Mietto L., Toffano G., Bigon E., Bruni A.,: Different responses of rodent mast cells to phosphatidylserine. Agents & Actions 14, 613-618 (1984)), that serine phospholipids have in vitro an activating effect on rodent mastocytes. The particular pharmacological behavior of natural PS, of which the effect of cerebral glucose increase is only one aspect, has important effects on the functions of the central nervous system. An activating influence was noticed on cholinergic and dopaminergic systems and a considerable ability in preserving the integrity of neuronal structures prone to deteriorate in old age. (Nunzi M.G., Milan F., Guidolin D., Toffano G.: Dendritic spine loss in hippocampus of aged rats. effect of brain phosphatidylserine administration. Neurobiology of Aging 8, 501-510 (1987)). All this results in an improvement of the learning and memorizing processes, which is particularly evident in older animals

(Calderini G. et al.: Pharmacological properties of phosphatidylserine in the aging brain: biochemical aspects and therapeutical potential. In: Phospholipids research and the Nervous System. L. Horrocks, L. Freysz and G. Toffano eds. Liviana Press pp. 233-241 (1986)).

Description of the Invention

We now have found new serine derivatives having the general formula (I)

(I) 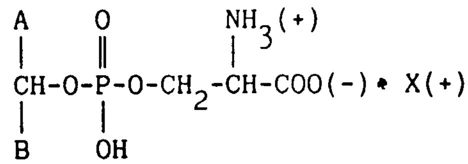

in which A = H $-(CH_2)_n-O-CO-R_1$, with n = 1, 2,
and B = $-(CH_2)m-O-CO-R_2$, with m = 1, 2, 3, 4 with the limitation that when A = $-(CH_2)_n-O-CO-R_1$, m = 1, 2 and in which $R_1$ and $R_2$, equal or different are radicals of acids of the aliphatic, aromatic, araliphatic, alicyclic or heterocylic series; $R_1$ and $R_2$ are preferably radicals of acids of the aliphatic series with a maximum of 20 carbon atoms, and which may be saturated or mono- or poly-unsatured, such as for instance formic, acetic, propionic, butyric, valerianic, capronic, caprinic, lauric, myristic, palmitic, palmitoleic, stearic, oleic, linoleic, linolenic, arachidonic acid; they may also represent radicals of hydroxy acids, such as lactic acid, of aminoacids such as glycine, or of bicarboxylic acids, such as succinic, glutaric, malonic or maleic acid.

When $R_1$ of $R_2$ are aromatic radicals, the preferred acids are the ones with only one benzene ring, in particular benzonic acid and its derivatives having as benzene ring substituents methyl, hydroxy, amino, or carboxyl groups, as for instance p-aminobenzoic acid, salicylic acid or phthalic acid.

X may be an alkali or earth-alkali metal, a primary, secundary, tertiary amine or a quaternary ammonium, aliphatic, aromatic or heterocyclic base.

X is preferably potassium, sodium, ammonium, calcium, magnesium, piperazine, proglycamine (3-amino-1,2-dihydroxy propane), lysine, meglumine (N-methyl glucamine), betaine, choline, aminobutanol, ethanolamine, arginine, carnitine, diethanolamine, dimethylamino ethanol, dimethyl piperazine.

The new derivatives according to the present invention are active in the therapy of human pathologies associated with a slow-down of cerebral metabolism and as hypolipidemizing agents.

The new serine derivatives according to the present invention were obtained employing a new synthesis process described hereinbelow.

General process outline

a) A phosphoric acid derivative of formula (II)

(II) 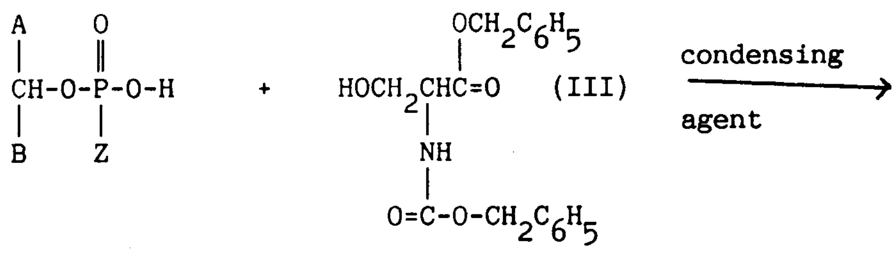


(wherein A = H, $-(CH_2-)_n$ $-O-CO-R_1$, n being 1 or 2,
and B= $-(CH_2-)_m-O-CO-R_2$, m being 1,2,3 or 4, with the limitation that when A = $-(CH_2)_n$ $-O-CO-R_1$, m is 1 or 2, and wherein $R_1$ and $R_2$, equal or different, are radicals of acids of the aliphatic, aromatic, araliphatic, alicyclic or heterocyclic series), and Z is H or OH N-carbobenzyloxy-L-serine benzyl ester (III) and an adeguate condensing agent are dissolved in a suitable organic solvent;
b) The thus obtained solution is heated to evaporate the solvent;

c) the residue is purified by crystallization from suitable solvents, obtaining a compound of formula (IV)

(IV)

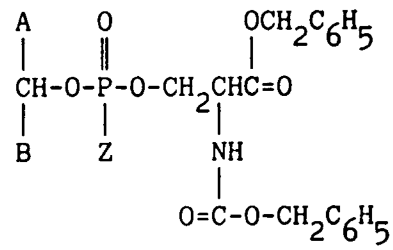

d) Compound (IV) is dissolved in a suitable organic solvent and hydrogenated in the presence of a hydrogenation catalyst; when Z = H, phase d) is modified thus:
the purified fraction is dissolved in a suitable organic solvent and oxidized with a suitable oxidizing agent, the preferred oxidizing agent being iodine in watery pyridine.
e) the solution is purified and concentrated until a precipitate forms which is separated and dried to obtain a compound of formula (V)

(V)

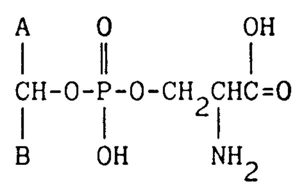

The preferred process conditions are:
in stage a) suitable organic solvents may be selected in the group consisting of tertiary organic bases, preferably pyridine or quinoline;
in stage b) heating is effected, for 10 to 60 minutes at a temperature of between approximately 40 ° C and 80 ° C, under stirring, leaving then the solution at room temperature for 2 to 3 hours;
in stage c), the residue is dried under reduced pressure and then taken up in ethyl- or isopropyl ether, the precipitate is separated, the ether phase washed with hydrochloric acid and water the solvent is evaporated;
in stage d), the preferred organic solvent is acetic acid and the hydrogenation is performed with the aid of palladium on carbon;
in stage e) the solution is filtered on Celite(R) or other similar filter aid and evaporated under reduced pressure.

Of the new carnitine derivatives according to the present invention, salts were prepared with metals and with organic bases, according to conventional methods.

Among the metal and organic bases salts are to be mentioned particularly the ones that can be employed therapeutically, such as the alkali and earth-alkali metal salts, for instance the potassium, sodium, ammonium, calcium, magnesium salts, and also the salts with organic bases for instance with primary, secundary, or tertiary amines or with aliphatic or aromatic or heterocyclic quaternary ammonium bases, such as piperazine, proglycamine (3-amino-1,2-dihydroxy propane), lysine, meglumine (N-methyl glucamine), betaine, choline, aminobutanol, ethanolamine, arginine, carnitine, diethanolamine, dimethylamino ethanol, dimethyl piperazine.

In order to demonstrate the therapeutic activity of the new serine derivatives according to the invention, we have performed in vivo and in vitro tests employing various phosphatidylserine derivatives obtained synthetically as described.

In particular, the effects on cerebral glucose levels and on histaminemia were studied, through evaluation of the desensibilization to natural lysophosphatidyl serine (Lyso PS) (in vivo experiments), and on the activation of rat mastocytes in the presence of New Growth Factor (NGF) (in vitro experiments).

The relationship between mastocyte activation and observed pharmacological effects in vivo is evident, no effect being observed in mice genetically free of mastocytes (Chang H.W., Inoue K., Bruni A., Boarato E., Toffano G.,: Stereoselective effects of lysophosphatidylserine in rodents. In press on Br. J. Pharmac.).

For the sake of clarity and brevity, the following initials will be used hereinafter:
PS-S1 for 1,3-dipalmitoylglycero-2-phosphoryl-L-serine;

PS-S2 for 1,3-dipivaloylglycero-2-phosphoryl-L-serine;
PS-S3 for 1,3-dimyristoylglycero-2-phosphoryl-L-serine.

Materials & methods

In vivo experiments

The products tested were PS-S1 and PS-S2.

All products were dispersed in 50 mM Tris (phosphate buffer) pH 7.8, by sonication at room temperature. The dispersions were administered endoveneously to male albino mice.

30 minutes after the injection the animals were sacrified to effect the cerebral glucose dosage.

In order to better evaluate in vivo the action of said products, repeated intraperitoneal administrations of the drugs, one per day for 4 days were made. On the $5^{th}$ day LysoPS was administered.

The desensitization which might have been induced by the new products would have resulted in a decrement of the LysoPs action.

In vitro experiments

The products were PS-S1, PS-S3. Peritoneal rat mastocytes were isolated and purified by the Lagunoff method (Lagunoff D.: The mechanism of histamine release from mast cells. Biochem. Pharmac. 21, 1889-1896 (1972)).

They were incubated with amounts of the products variable between 0.1 and 125 uM of the products in the presence of 5-10 ng NGF (Nerve Growth Factor), as this factor enhances the mastocyte activation produced by serine phospholipids (Bruni A., Bigon E., Boarato E., Mietto L., Leon A., Toffano G.: Interaction between nerve growth factor and lysophosphatidylserine on rat peritoneal mast cells. FEBS Letters 138, 190-192 (1982)).

The composition of the incubation medium and the operative process are described in Boarato E., Mietto L., Toffano G., Bigon E., Bruni A.: Different responses of rodent mast cells to phosphatidylserine. Agents & Actions 14, 613-618 (1984).

Analytical methods

Cerebral glucose was measured by the enzymatic method, following the conditions described by Bigon E., Boarato E., Bruni A., Leon A., Toffano G.: Pharmacological effects of phosphatidylserine liposomes: regulation of glycolysis and energy level in brain. Br. J. Pharmac. 66, 167-174 (1979).

Histamine was measured by the fluorometric method described by Shore P.A., Burkhalter A., Cohn V.H.: A method for the flourimetric assay of histamine in tissues. J. Pharmac. Exp. Ther. 127, 182-186 (1959) or by the radio immunological method using the Immunotech radioimmunoassay kit.

Results

Table 1 shows the PS-S1 and PS-S2 action on cerebral glucose contents. The dispersion of the various compounds in 50 mM Tris, pH 7.8, was injected endovenously to 25-30 g mices. Cerebral glucose was determined after 30 minutes. The values reported are the average of 5 mice.

TABLE 1

| Product | Dose (mg/kg e.v.) | Cerebral glucose (µmol/g fresh tissue) |
|---|---|---|
| Control | Tris 350 µl/mouse | 1.1 |
| PS-S1 | 10<br>20<br>40 | 3.15<br>2.9<br>1.3 |
| PS-S2 | 10<br>20<br>40 | 1.5<br>1.5<br>1.6 |

Table 1 shows an evident increase in cerebral glucose, particularly by the administration of PS-S1 at 10 mg/kg. The decreasing effect at higher doses is due to the rapid desensibilization in the presence of high drug amounts in the organism.

In order to obtain a better evaluation of the in vivo action of the product, the desensibilization to LysoPs induced following repeated intraperitoneal administrations to the mouse of natural PS and PS-1 was also evaluated. The animals (w. 35 g) were treated for 4 days with the phospholipids (50 mg/kg i.p.).

On the 5$^{th}$ day Lyso PS was injected endovenously (5 mg/kg). Histaminemia was measured 30 minutes after the Lyso PS injection. Averages ± a.s.e. from 10 mice. The data are reported in Table 2.

TABLE 2

| Product | Dose (mg/kg) | Administration | Histaminemia (ng/ml plasma) |
|---|---|---|---|
| Control<br>LysoPS | Tris 350 µl/mouse<br>5 | <br>e.v. | 78<br>940 |
| PS + LysoPS | 50<br>5 | i.p. (x 4 die)<br>e.v. (5° die) | 480 |
| PS-S1 + LysoPS | 50<br>5 | i.p. (x 4 die)<br>e.v. (5° die) | 550 |

Table 2 shows that PS-S1, similarly to natural PS, produces in the mouse desensibilization to LysoPS, however to a lower extent.

Table 3 shows the in vitro effect of natural LysoPS and of synthetic PS-S1 and PS-S3 on the activation of isolated rat mastocytes in the presence of 5-10 ng NGF. The incubation technique and the mastocyte preparation are described by Boarato E., Mietto L., Toffano G., Bigon E., Bruni A.: Different responses of rodent mast cells to phosphatidylserine. Agents & Actions 14, 613-618 (1984).

Mastocyte activation was deduced from the histamine secretion calculated as percentage of the total contents found in the added cells: 4-5 µg/3x10$^5$ cells.

The data reported in the following Table evidence the histamine increase induced by the new serine derivatives according to the present invention.

TABLE 3

| Product | Concentration (μM) | Histamine release (%) |
|---|---|---|
| Control | ---- | 5 |
| PS-S1 | 5<br>12.5<br>62.5<br>125 | 5<br>5<br>8<br>38 |
| PS-S3 | 6<br>120 | 10.3<br>12 |

Conclusions

The obtained data show that the new derivatives are active both in vivo (increase of cerebral glucose and desensibilization induction) and in vitro (histamine release from isolated rat mastocytes) similarly to natural PS.

We can conclude therefore that the new serine derivatives according to the present invention, showing the same pharmacological activity as natural PS, are active in the pathological conditions typical of old age, in the Alzheimer disease, loss of memory and senile dementia in general, anoxic and oedematic situations in general, cerebral fatigue, neuroendocrine and immunologic alterations (e.g. stress, depression, anxiety).

For illustrative purposes only, we give some detailed examples of processes for preparing the compounds according to the present invention, performed following the general outline given supra.

EXAMPLE 1

1,3-dipalmitoyl glycero-2-phosphoryl-L-Serine

a) To 150 ml pyridine, 7.5 g dipalmitoylglycero-2-phosphoric acid (prepared according to H-Eibl and A. Blume. Biochim. Biophys. Acta 553, 476-488 (1979), 5.3 g N-carbobenzyloxy-L-Serine benzyl ester (prepared according to E. Baer & J. Maurukas: J. Biol. Chem 212, 25-38 g (1955)) and 8.8 g 2,4,6 triisopropylbenzene sulphonyl chloride (TPS), as condensing agent are added under stirring at 70 °C for 10 minutes. The mixture is left standing at room temperature for 3 hours.

Pyridine is evaporated under reduced pressure and the residue, dried as thoroughly as possible, is taken up in 600 ml ethyl ether; an immediate formation takes place of a crystalline white phase consisting of TPS, which is filtered out. The ether phase is washed with 0.1 N HCl and water. The ethyl ether is evaporated and the residue is crystallized twice from methanol. 8.2 g 1,3-dipalmitoylglycero - 2 - phosphoryl - (N-carbobenzyloxy)-L-serine benzyl ester are obtained having the following characteristics:

Thin layer chromatography:

$CHCl^3$: $CH^3OH$ (9:1)- Rf = 0.2

M.P. = 149-151 °C.

b) 8 g 1,3-dipalmitoyl-glycero-2-phosphoryl-(N-carbobenzyloxy)-L-serine benzyl ester, are taken up in 700 ml glacial acetic acid.

After heating slightly, 5 g palladium 10% on carbon are added and hydrogenetion is carried out under normal pressure keeping the temperature at 20-30 °C. The maximum hydrogen absorption takes place after 4 hours; after heating to 40 °C and filtration on Celite (R), 400 ml 2:1 chloroform: methanol are added and then 600 ml water.

The lower layer is separated, which is washed further with water, which is added to the main solution. By concentration to reduced volume a precipitate is obtained which is filtered and dried under high vacuum. 4 g 1,3-dipalmitoyl-glycero-2-phosphoryl-L-serine are obtained with a 64% yield. The thus obtained product is crystallizable from hot dioxane and acetic acid and the thin layer chromatography shows:

$CHCl^3$:$CH^3OH$:$H^2O$ (60:30:4)

Rf = 0.25

EXAMPLE 2

1.3-dipivaloyl-glycero-2-phosphoryl-L-serine

10g 1,3-dipivaloylglycero-2-phosphoric acid [prepared from 2-O-benzylglycerol (obtained according to Verkade et al.: Recueil Trav. Chim. Pays Bas 61,836 (1940), by reaction with pivaloyl chloride, debenzylation of the 1,3-dipivaloyl-glycero-2-benzyl ether and successive phosphorylation with $POCl^3$], 10 g N-carbobenzyloxy-L-serine benzyl ester (prepared according to R. Baer and J. Maurukas: J. Biol. Chem. 212,25-38 (1955) and 19 g 2,4,6-tri-isopropyl-benzene sulphonylchloride (TPS) are dissolved under stirring in 200 ml pyridine at 70 °C for 30 minutes. After 3 hours standing at room temperature, pyridine is evaporated under reduced pressure, and the residue, dried as thoroughly as possible, is taken up in 600 ml ethyl ether; immediate formation of a white precipitate takes place, which is filtered off. The ether phase is washed with 0.5 N HCl and water.

After ether evaporation, 19 g 1.3-dipivaloyl-glycero-2-phosphoryl-(N-carbobenzyloxy)-L-serine benzyl ester are obtained; thin layer chromatography gives the following results:

$CHCl^3$:$CH^3OH$ (7:3)

Rf = 0.6

b) 10 g 1,3-dipivaloyl-glycero-2-phosphoryl-N-carbobenzyloxy-L-serinebenzyl ester are taken up in 700 ml glacial acetic acid 6 g 10% palladium on carbon are added.

Hydrogenation takes place at room temperature and normal pressure under vigorous stirring. The hydrogen absorption ends after 4 hours; after filtration on Celite (R), acetic acid is evaporated. The phospholipid is purified by chromatography on PREP L. 500/A WATERS. The purified solution is dried under high vacuum and the residue washed with acetone.

3.5 g 1,3 dipivaloyl-glycero-2-phosphoryl-L-serine are obtained.

Thin layer chromatography shows:

$CHCl^3$:$CH^3$ OH:$H^2$ O (60:30:4)

Rf = 0.13

EXAMPLE 3

1,3 dimyristoyl-glycero-2-phosphoryl-L-serine

Following a procedure similar to the one of Example 2, 1,3-dimyristoyl-glycero-2-phosphoryl-L-serine is obtained; thin layer chromathophy shows:

$CHCl^3$:$CH^3OH$: $H^2O$ (60:30:4)

Rf = 0.24

EXAMPLE 4

1-mirysthoyl-2-deoxyglycero-3-phospho-L-serine

a) 55.7 g imidazole are dissolved in 500 ml hot toluene. Toluene is evaporated and the residue, taken up with 500 ml methylene chloride, is transferred in a 2000 ml reaction vessel fitted with a stirrer.

After cooling to -15 °C, 32.1 g phosphor trichloride dissolved in 350 ml methylene chloride are added. 47.3 g triethylamine in 350 ml methylene chloride are then added, and, after 10 minutes, 17 g monomirysthoyl-1,3-propanediol dissolved in 500 ml methylene chloride. Once the addition is terminated, the temperature is brought to -5 °C and after 1 hour to room temperature. After further 30 minutes, the solvent is evaporated, the residue is taken up in 100 ml acetone, the solution is cooled to -5 °C and, under stirring, 1200 ml of a 5% sodium bicarbonate water solution are added.

After 1 hour the precipitate is filtered and washed twice with 500 ml water and twice with 500 ml acetone. After a further washing with 500 ml warm ethyl ether, 21 g 1-mirysthoyl-2-deoxyglycero-3-H-phosphonate are obtained.

18 g 1-mirysthoyl-2-deoxy glycero-3-H-phosphonate and 21.3 g N-(terbutoxy carbonyl)-L-serine are dissolved in 500 ml anhydrous pyridine. After concentration, the residue is taken up in 800 ml anhydrous pyridine, 28.8 g 5,5-dimethyl-2-oxo-2-chloro-1,3,2-dioxophosphorinane (McConnell R.L., Coover H.W., J. Org. Chem., 1959, 24, 630-635) and the reaction is carried out at 40 °C, under continuous stirring for 30 minutes.

Pyridine is evaporated and the residue is dried under high vacuum. The dried residue is taken up in 1000 ml ethyl ether under stirring for 30 minutes, then the salt is filtered out and the solution is concentrated.

400 ml pyridine are added and then 10 ml water and 26.4 g jodine; after stirring for 15 minutes, 1000 ml chloroform and 1000 ml 5% sodium bisulphite water solution are added.

After separation, the washing is repeated with 500 ml chloroform. Chloroform is dried and concentrated. The residue is taken up in 1000 ml acetone, 160 ml 1M water solution of sodium acetate are added, and, after 1 hour stirring at -15 °C is filtered and dried under high vacuum. 17 g 1-mirysthoyl-2-deoxy glycero-3-phospho-N-(terbuthoxy carbonyl)-L-serine are obtained.

c) 16 g 1-mirystoyl-2-deoxy-glycero-3-phospho-N-(terbuthoxycarbonyl)-L-serine suspended in 250 ml methylene chloride are added, drop by drop, to a mixture (250 ml) trifluor acetic acid and (250 ml) percloric acid 70%, under stirring at 0 °C temperature.

The solution in left 30 minutes and diluted with 600 ml chloroform, 600 ml water and 300 ml methanol.

After slowly adding 1200 ml sodium carbonate water solution 0,5 M, it is separated.

It is washed for two times with 300 ml chloroform and the solution is concentrated.

After precipitation with 1000 ml methanol, the solid part is washed with acetone.

The residue is dried under high vacuum.

8 g 1-mirystoyl-2-deoxyglycero-3-phospho-L-serine are obtained.

Thin layer chromatography: eluent chlorophorm/methanol/water (60:30:4); Rf: 0.10.

EXAMPLE 5

1,3-dimirysthoylglycero-2-phospho-L-serine

a) 55.7 g imidazole are dissolved in 500 ml hot toluene. Toluene is evaporated and the residue, taken up with 500 ml methylene chloride, is transferred in a 2000 ml reaction vessel fitted with a stirrer.

After cooling to -15 °C, 32.1 g phosphor trichloride dissolved in 350 ml methylene chloride are added. 47.3 g triethylamine in 350 ml methylene chloride are then added, and, after 10 minutes, 30 g 1,3-dimirystoylglycerol dissolved in 500 ml methylene chloride.

Once the addition is terminated, the temperature is brought to -5 °C and after 1 hour to room temperature. After further 30 minutes, the solvent is evaporated, the residue is taken up in 100 ml acetone, the solution is cooled to -5 °C and, under stirring, 1200 ml of a 5% sodium bicarbonate water solution are added.

After 1 hour the precipitate is filtered and washed twice with 500 ml water and twice with 500 ml acetone. After a further washing with 500 ml warm ethyl ether, 31 g 1,3-dimirystoylglycero-2-H-phosphonate are obtained.

Thin layer chromatography: chloroform/methanol/water eluent (60:30:4); Rf: 0.58.

b) 30 g 1,3-dimirystoylglycero-2-H-phosphonate and 21.3 g N-(terbutoxycarbonyl)-L-serine are dissolved in 500 ml anhydrous pyridine. After concentration, the residue is taken up in 800 ml anhydrous pyridine, 28.8 g 5,5-dimethyl-2-oxo-2-chloro-1,3,2-dioxophosphorinane (McConnell R.L., Coover H.W., J. Org. Chem., 1959, 24, 630-635) and the reaction is carried out at 40 °C, under continuous stirring for 30 minutes.

Pyridine is evaporated and the residue is dried under high vacuum. The dried residue is taken up in 1000 ml ethyl ether under stirring for 30 minutes, then the salt is filtered out and the solution is concentrated.

400 ml pyridine are added and then 10 ml water and 26.4 g jodine; after stirring for 15 minutes, 1000 ml chloroform and 1000 ml 5% sodium bisulphite water solution are added.

After separation, the washing is repeated with 500 ml chloroform. Chloroform is dried and concentrated. The residue is taken up in 1000 ml acetone, 160 ml 1M water solution of sodium acetate are added, and, after 1 hour stirring at -15 °C is filtered and dried under high vacuum. 34 g 1,3-dimirystoylglycero-2-phospho-N-(terbutoxycarbonyl)-L-serine are obtained. M.W.: 801.98-$C^{39}H^{73}O^{12}P$ N Na.

Thin layer chromatography: chloroform/methanol/water (60:30:4) eluent, Rf: 0.30.

c) 25 g 1,3-dimirystoylglycero-2-phospho-N-(terbutoxycarbonyl)-L-serinesuspended in 250 ml chloride methylene are added, drop by drop, to a mixture (250 ml) of trifluoro acetic acid and (250 ml) perchloric acid 70%, under stirring at 0 °C temperature.

The solution is left 30 minutes and diluted with 600 ml chloroform, 600 ml water and 300 ml methanol.

After slowly adding 1200 ml sodium carbonate water solution 0,5 M, it is separated.
It is washed for two times with 300 ml chloroform and the solution is concentrated.
After precipitation with 1000 ml methanol, the solid part is washed with acetone.
The residue is dried under high vacuum.
18 g 1,3-dimirystoyl-glycero-2-phospho-L-serine are obtained.
Thin layer chromatography: eluent chlorophorm/methanol/water (60:30:4); Rf: 0.28

EXAMPLE 6

1,3-dipalmitoylglycero-2-phospho-L-serine

a) 50.5 g imidazole are dissolved in 450 ml hot toluene. Toluene is evaporated and the residue, taken up with 450 ml methylene chloride, is transferred in a 2000 ml reaction vessel fitted with a stirrer.

After cooling to -15°C, 29.1 g phosphor trichloride dissolved in 350 ml methylene chloride are added. 42.9 g triethylamine in 350 ml methylene chloride are then added, and, after 10 minutes, 30 g 1,3-dipalmitoylglycerol dissolved in 600 ml methylene chloride.

Once the addition is terminated, the temperature is brought to -5°C and after 1 hour to room temperature. After further 30 minutes, the solvent is evaporated, the residue is taken up in 100 ml acetone, the solution is cooled to -5°C and, under stirring, 1000 ml of a 5% sodium bicarbonate water solution are added.

After 1 hour the precipitate is filtered and washed twice with 500 ml water and twice with 500 ml acetone. After a further washing with 500 ml warm ethyl ether, 32 g 1,3-dipalmitoylglycero-2-H-phosphonate are obtained.

Thin layer chromatography: chloroform/methanol/water eluent (60:30:4); Rf: 0.60.

b) 30 g 1,3-dipalmitoylglycero-2-H-phosphonate and 19.3 g N-(terbutoxycarbonyl)-L-serine are dissolved in 500 ml anhydrous pyridine. After concentration, the residue is taken up in 800 ml anhydrous pyridine, 26 g 5,5-dimethyl-2-oxo-2-chloro-1,3,2-dioxophosphorinane (McConnell R.L., Coover H.W., J. Org. Chem., 1959, 24, 630-635) and the reaction is carried out at 40°C, under continuous stirring for 30 minutes.

Pyridine is evaporated and the residue is dried under high vacuum for 30 minutes. The dried residue is taken up in 1000 ml ethyl ether under stirring for 30 minutes, then the salt is filtered out and the solution is concentrated.

400 ml pyridine are added and then 10 ml water and 23.8 g jodine; after stirring for 15 minutes, 1000 ml chloroform and 900 ml 5% sodium bisulphite water solution are added.

After separation, the washing is repeated with 500 ml chloroform. Chloroform is dried and concentrated. The residue is taken up in 1000 ml acetone, 150 ml 1M water solution of sodium acetate are added, and, after 1 hour stirring at -15°C is filtered and dried under high vacuum. 35 g 1,3-dipalmitoylglycero-2-phospho-N-(terbutoxycarbonyl)-L-serine are obtained.

Thin layer chromatography: chloroform/methanol/water (60:30:4) eluent, Rf: 0.31.

c) 30 g 1,3-dipalmitoylglycero-2-phospho-N-(terbutoxycarbonyl)-L-serinesuspended in 250 ml chloride methylene are added, drop by drop, to a mixture (250 ml) of trifluoro acetic acid and (250 ml) perchloric acid 70%, under stirring at 0°C temperature.
The solution is left 30 minutes and diluted with 600 ml chloroform, 600 ml water and 300 ml methanol.
After slowly adding 1200 ml sodium carbonate water solution 0,5 M, it is separated.
It is washed for two times with 300 ml chloroform and the solution is concentrated.
After precipitation with 1000 ml methanol, the solid part is washed with acetone.
The residue is dried under high vacuum.
19 g 1,3-dipalmitoyl-glycero-2-phospho-L-serine are obtained.
Thin layer chromatography: eluent chlorophorm/methanol/water (60:30:4); Rf: 0.28.

EXAMPLE 7

1,3-distearoylglycero-2-phospho-L-serine

a) 45.8 g imidazole are dissolved in 400 ml hot toluene. Toluene is evaporated and the residue, taken up with 400 ml methylene chloride, is transferred in a 2000 ml reaction vessel fitted with a stirrer.

After cooling to -15°C, 19.8 g phosphor trichloride dissolved in 300 ml methylene chloride are added. 38.9 g triethylamine in 300 ml methylene chloride are then added, and, after 10 minutes, 30 g

1,3-distearoylglycerol dissolved in 500 ml methylene chloride.

Once the addition is terminated, the temperature is brought to -5°C and after 1 hour to room temperature. After further 30 minutes, the solvent is evaporated, the residue is taken up in 100 ml acetone, the solution is cooled to -5°C and, under stirring, 800 ml of a 5% sodium bicarbonate water solution are added.

After 1 hour the precipitate is filtered and washed twice with 500 ml water and twice with 500 ml acetone. After a further washing with 500 ml warm ethyl ether, 31 g 1,3-distearoylglycero-2-H-phosphonate are obtained.

Thin layer chromatography: chloroform/methanol/water eluent (60:30:4); Rf: 0.61.

b) 30 g 1,3-distearoylglycero-2-H-phosphonate and 17.6 g N-(terbutoxycarbonyl)-L-serine are dissolved in 500 ml anhydrous pyridine. After concentration, the residue is taken up in 800 ml anhydrous pyridine, 23.8 g 5,5-dimethyl-2-oxo-2-chloro-1,3,2-dioxophosphorinane (McConnell R.L., Coover H.W., J. Org. Chem., 1959, 24, 630-635) and the reaction is carried out at 40°C, under continuous stirring for 30 minutes.

Pyridine is evaporated and the residue is dried under high vacuum for 30 minutes. The dried residue is taken up in 1000 ml ethyl ether under stirring for 30 minutes, then the salt is filtered out and the solution is concentrated.

400 ml pyridine are added and then 10 ml water and 21.8 g jodine; after stirring for 15 minutes, 1000 ml chloroform and 850 ml 5% sodium bisulphite water solution are added.

After separation, the washing is repeated with 500 ml chloroform.

Chloroform is dried and concentrated. The residue is taken up in 1000 ml acetone, 130 ml 1M water solution of sodium acetate are added, and, after 1 hour stirring at -15 °C is filtered and dried under high vacuum. 34 g 1,3-distearoylglycero-2-phospho-N-(terbutoxycarbonyl)-L-serine are obtained.

Thin layer chromatography: chloroform/methanol/water (60:30:4) eluent, Rf: 0.33.

c) 30 g 1,3distearoylglycero-2-phospho-N-(terbutoxycarbonyl)-L-serine suspended in 250 ml chloride methylene are added, drop by drop, to a mixture (250 ml) of trifluoro acetic acid and (250 ml) perchloric acid 70%, under stirring at 0°C temperature.

The solution is left 30 minutes and diluted with 600 ml chloroform, 600 ml water and 300 ml methanol.

After slowly adding 1200 ml sodium carbonate water solution 0,5 M, it is separated.

It is washed for two times with 300 ml chloroform and the solution is concentrated.

After precipitation with 1000 ml methanol, the solid part is washed with acetone.

The residue is dried under high vacuum.

18 g 1,3-distearoyl-glycero-2-phospho-L-serine are obtained.

Thin layer chromatography: eluent chlorophorm/methanol/water (60:30:4); Rf;0.29

Following the general process outline described, one can obtain all the compounds comprised in general formula (I).

The active principles according to the present invention may be employed for the preparation of pharmaceutical compositions which are effective in the pathological situations mentioned, particularly for the preparation of pharmaceutical compositions which can be administerd orally or parenterally, containing an amount of active principle comprised between 20 and 1000 mg, preferably between 40 and 350 mg, in association with the excipients normally employed in the pharmaceutical technique.

The therapeutic method for the therapy of human pathologies due to neuronal damages, characterized by the administration of a therapeutically effective amount of phosphatidyl carnitine derivatives, or of their pharmacologically acceptable salts of general formula (I) is effected by the administration, per os or parenteral, of an amount of active principle comprised between 10 and 80 mg/kg/day, preferably between 20 and 50 mg/kg/day in from 2 to 4 administration/day.

The injectable pharmaceutical compositions according to the present invention are employed preferably in emergency cases of central nervous system pathology, more specifically, these pharmaceutical compositions may be employed in the emergency therapy of anoxic cerebropathies, traumatic syndromes, senile and pre-senile dementia and metabolic encephalopathies.

The pharmaceutical compositions according to the present invention suitable for oral administration may preferably be employed in chronic therapies; more specifically these pharmaceutical compositions may be employed in the chronic therapy of psychomotor senile involutive syndromes, chronic vascular cerebropathies, old age metabolic encephalopathies, senile dementia syndromes and pre-senile syndromes also as a result of traumas, post-anoxic cerebropathies and extrapyramidal syndromes.

For illustrative purposes only we report hereinbelow some examples of pharmaceutical compositions prepared according to the present invention.

INJECTABLE PHARMACEUTICAL COMPOSITIONS

EXAMPLE 1

A 2 ml vial contains:

| | |
|---|---|
| 1,3-dipalmitoyl glycero-2-phosphoryl-L-serine | 40.0 mg |
| Monobasic sodium phosphate | 2.4 mg |
| Bibasic sodium phosphate | 2.26 mg |
| bidistilled apyrogenic water q.s. to | 2 ml |

EXAMPLE 2

A 2 ml vial contains:

| | |
|---|---|
| 1,3-dipivaloyl-glycero-2-phosphoryl-L-serine | 40.0 mg |
| Monobasic sodium phosphate | 2.4 mg |
| Bibasic sodium phosphate | 2.26 mg |
| bidistilled apyrogenic water q.s. | 2 ml |

EXAMPLE 3

A 2 ml vial contains:

| | |
|---|---|
| 1,3-dimyristoyl-glycero-2-phosphoryl-L-serine | 40.0 mg |
| Monobasic sodium phosphate | 2.4 mg |
| Bibasic sodium phosphate | 2.26 mg |
| bidistilled apyrogenic water q.s. to | 2 ml |

EXAMPLE 4

A 3 ml vial contains:

| | |
|---|---|
| 1,3-dipalmitoyl-glycero-2-phosphoryl-L-serine | 80.0 mg |
| Monobasic sodium phosphate | 3.21 mg |
| Bibasic sodium phosphate | 3.39 mg |
| Mannitol | 30 mg |
| bidistilled apyrogenic water q.s. to | 3 ml |

EXAMPLE 5

A 3 ml vial contains:

| | |
|---|---|
| 1,3-dipivaloyl-glycero-2-phosphoryl-L-serine | 80.0 mg |
| Monobasic sodium phosphate | 3.21 mg |
| Bibasic sodium phosphate | 3.39 mg |
| Mannitol | 30 mg |
| bidistilled apyrogenic water q.s. to | 3 ml |

EXAMPLE 6

A ml 3 vial cointains:

| | |
|---|---|
| 1,3-dimyristoyl-glycero-2phosphoryl-L-serine | 80.0 mg |
| Monobasic sodium phosphate | 3.21 mg |
| Bibasic sodium phosphate | 3.39 mg |
| Mannitol | 30 mg |
| Bidistilled apyrogenic er q.s. to | 3 ml |

Oral pharmaceutical compositions

EXAMPLE 7

A gelatine capsule contains:

| | |
|---|---|
| 1,3-dipalmitoyl-glycero-2-phosphoryl-L-serine | 120 mg |
| Vegetable oil | 270 mg |
| Bee wax | 1 mg |

EXAMPLE 8

A gelatine capsule contains:

| | |
|---|---|
| 1,3-dipivaloyl-glycero-2-phosphoryl-L-serine | 120 mg |
| Vegetable oil | 270 mg |
| Bee wax | 1 mg |

EXAMPLE 9

A gelatine capsule contains:

| | |
|---|---|
| 1,3-dimyristoyl-glycero-2-phosphoryl-L-serine | 120 mg |
| Vegetable oil | 270 mg |
| Bee wax | 1 mg |

EXAMPLE 10

A gelatine capsule contains:

| | |
|---|---|
| 1,3-dipalmitoyl-glycero-2-phosphoryl-L-serine | 320 mg |
| Vegetable oil | 270 mg |
| Bee wax | 1 mg |

EXAMPLE 11

A gelatine capsule contains:

| | |
|---|---|
| 1,3-dipivaloyl-glycero-2-phosphoryl-L-serine | 320 mg |
| Vegetable oil | 270 mg |
| Bee wax | 1 mg |

EXAMPLE 12

A gelatine capsule contains:

| | |
|---|---|
| 1,3-dimyristoyl-glycero-2-phosphoryl-L-serine | 320 mg |
| Vegetable oil | 270 mg |
| Bee wax | 1 mg |

EXAMPLE 13

A dragee contains:

| | |
|---|---|
| 1,3-dipalmitoyl-glycero-2-phosphoryl-L-serine | 60 mg |
| Mannitol | 100 mg |
| Microcrystalline cellulose | 25 mg |
| Starch | 5 mg |
| Sucrose | 30 mg |
| Lacquer | 5 mg |

EXAMPLE 14

A dragee contains:

| | |
|---|---|
| 1,3-dipivaloyl-glycero-2-phosphoryl-L-serine | 60 mg |
| Mannitol | 100 mg |
| Microcrystalline cellulose | 25 mg |
| Starch | 5 mg |
| Sucrose | 30 mg |
| Lacquer | 5 mg |

EXAMPLE 15

A dragee contains:

| | |
|---|---|
| 1,3-dimyristoyl-glycero-2-phosphoryl-L-serine | 60 mg |
| Mannitol | 100 mg |
| Microcrystalline cellulose | 25 mg |
| Starch | 5 mg |
| Sucrose | 30 mg |
| Lacquer | 5 mg |

EXAMPLE 16

A cachet contains:

| | |
|---|---|
| 1,3-dipalmitoyl-glycero-2-phosphoryl-L-serine<br>Mannitol<br>Lactose | 185 mg<br>100 mg<br>100 mg |

EXAMPLE 17

A cachet contains:

| | |
|---|---|
| 1,3-dipivaloyl-glycero-2-phosphoryl-L-serine<br>Mannitol<br>Lactose | 185 mg<br>100 mg<br>100 mg |

EXAMPLE 18

A cachet contains:

| | |
|---|---|
| 1,3-dimyristoyl-glycero-2-phosphoryl-L-serine<br>Mannitol<br>Lactose | 185 mg<br>100 mg<br>100 mg |

**Claims**
**Claims for the following Contracting States : AT, BE, CH, DE, DK, FR, GB, IT, LI, LU, NL, SE**

**1.** Serine derivatives of general formula (I)

(I) 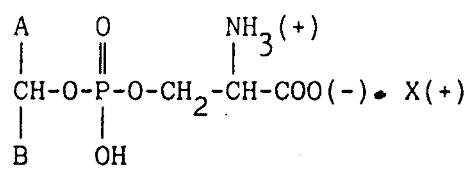

wherein A = H, - $(CH_2)_n$ - O-CO-$R_1$ n = 1 or 2,
and B = $(CH_2)_m$ - O-CO-$R_2$, m = 1, 2, or 4 with the limitation that, when A = -$(CH_2)_n$ - O-CO-$R_1$, m = 1 or 2, and wherein $R_1$ and $R_2$, equal or different, are radicals of acids of the aliphatic, aromatic, araliphatic, alicyclic, or heterocyclic series, X being an alkali or earth-alkali metal, a primary, secundary, tertiary amine or a quaternary ammonium, aliphatic, aromatic or heterocyclic base.

**2.** Serine derivatives according to claim 1, characterized in that $R_1$ and $R_2$ are radicals of the aliphatic saturated, mono- or polyunsaturated acids of the aliphatic series with a maximum of 20 C atoms.

**3.** Serine derivatives according to claim 1, characterized in that $R_1$ and $R_2$ are radicals of pivalic, lauric, myristic, palmitic, palmitoleic, stearic, oleic, linoleic, linolenic or arachidonic acid.

**4.** Serine derivatives according to claim 1, characterized in that $R_1$ and $R_2$ are hydroxyacid radicals.

**5.** Serine derivatives according to claim 1, characterized in that $R_1$ and $R_2$ are aminoacid radicals.

**6.** Serine derivatives according to claim 1, characterized in that $R_1$ and $R_2$ are radicals of benzoic acid which may be substituted on the benzene ring.

**7.** Serine derivatives according to claim 1, characterized in that $R_1$ and $R_2$ are bicarboxylic acid radicals.

**8.** Process for the preparation of serine derivatives of general formula (I)

(I) 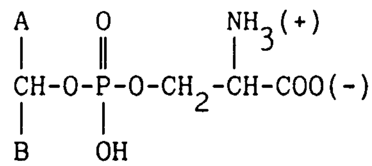

wherein A = H, $-(CH_2)_n$ -O -CO-$R_1$, n = 1 or 2,
and B = $-(CH_2)_m$ -O -CO-$R_2$, m = 1, 2, 3 or 4 with the limitation that, when A = $-(CH_2)_n$ - O-CO-$R_1$, m = 1 or 2, and wherein $R_1$ and $R_2$, equal or different, are radicals of acids of the aliphatic, aromatic, araliphatic, alicyclic, heteroyclic series,and Z is H or OH, comprising reacting a phosphoric acid derivative of formula (II)

(II) 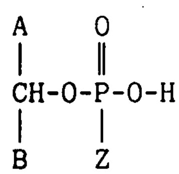

wherein A and B have the defined meanings, with N-carbobenzyloxy-L-serine benzyl ester, in the presence of a condensing agent and catalytically hydrogenating the obtained compound (IV)

(IV) 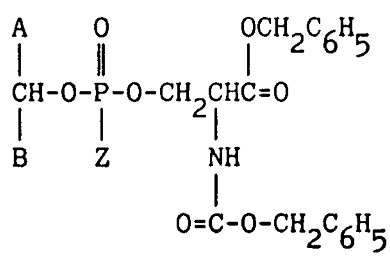

in which A and B have the previously defined meanings.

**9.** Process according to claim 8 characterized by:
a) reacting a phosphoric acid derivative of formula (II); wherein A,B,$R_1$ and $R_2$ have the above defined meanings, with N-carbobenzyloxy-L-serine-benzyl ester (III) in the presence of a condensing agent and of an inert organic solvent:

(II) 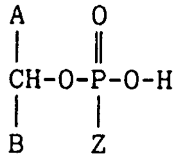 + 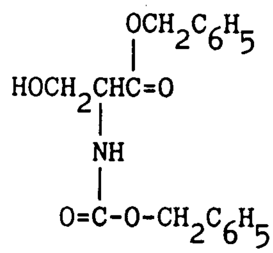 (III)

b) heating the obtained solution to evaporate the solvent

c) taking up the residue in a suitable organic solvent and purifying the solution, obtaining a compound of formula (IV)

(IV) 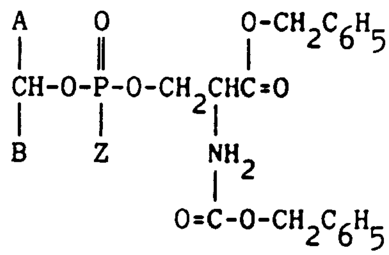

d) dissolving compound (IV) in a suitable organic solvent and hydrogenating it in the presence of a hydrogenation catalyst; when Z = H, phase d) is modified thus:

the purified fraction is dissolved in a suitable organic solvent and oxidized with a suitable oxidizing agent, the preferred oxidizing agent being iodine in watery pyridine.

e) purifying and concentrating the solution;

f) drying the obtained precipitate obtaining a compound of formula (V)

(V) 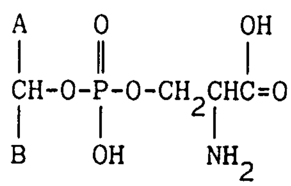

wherein A and B are as previously defined.

**10.** Process according to claim 9, characterized in that:

in stage a) suitable organic solvents may be selected from the group consisting of tertiary organic bases, preferably pyridine and quinoline;

in stage b) the heating is done first for a time of between approximately 10 and 60 minutes at a temperature between approximately 40° and approximately 80°C, under stirring and thereafter for a time of between 2 and 3 hours at room temperature;

in stage c) the residue is dried under reduced pressure, taken up in ethyl- or isopropyl ether, the precipitate is filtered off, the ether phase is washed with HCl and water and the solvent evaporated:

in stage d) the preferred organic solvent is acetic acid and the hydrogenation is performed with the aid of palladium on carbon;

in stage e) the solution is filtered on Celite (R) or on other filter aid and evaporated under reduced pressure.

**11.** Pharmaceutical compositions containing as active principle at least one compound of general formula (I)

(I) 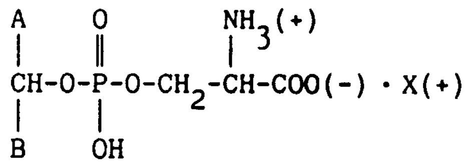

wherein A = H, $-(CH_2)_n$ -O-CO-$R_1$, n = 1 or 2,
and B = $(CH_2)_m$-O-CO-$R_2$- m = 1,2, 3 or 4 with the limitation that, when A = $-(CH_2)_n$ -O-CO-$R_1$, m = 1 or 2, and in which $R_1$ and $R_2$, equal or different, are radicals of acids of the aliphatic, aromatic, araliphatic, alicyclic, heteroyclic series, X may be an alkali or earth-alkali metal, a primary, secundary, tertiary amine or a quaternary ammonium, aliphatic, aromatic or heterocyclic base, active in the therapy of human pathologies associated with a slowing down of the cerebral metabolism and as hypolipidemizing agents.

**12.** Pharmaceutical compositions according to claim 11, characterized in that $R_1$ and $R_2$ are radicals of aliphatic, saturated, mono- or polyunsaturated acids with a maximum of 20 carbon atoms, and preferably radicals of the acids: pivalic, lauric, myristic, palmitic, palmitoleic, stearic, oleic, linoleic, linolenic or arachidonic.

**13.** Pharmaceutical compositions according to claim 11, characterized in that $R_1$ and $R_2$ are radicals of hydroxyacids, aminoacids, bicarboxylic acids, simple or substituted aromatic acids.

**14.** Pharmaceutical compositions according to claim 11, characterized in that they are active against human pathologies typical of old age, Alzheimer disease, loss of memory and senile dementia in general, anoxic and oeadematic situations in general, cerebral fatigue, neuroendocrine and immunologic alterations.

**15.** Pharmaceutical compositions according to claim 11, characterized in that the active principle contents is between 20 and 1000 mg.

**16.** Pharmaceutical compositions according to claim 15, characterized in that the active principle contents is between 40 and 350 mg.

**Claims for the following Contracting States : ES, GR**

**1.** Process for the preparation of serine derivatives of general formula (I)

(I) 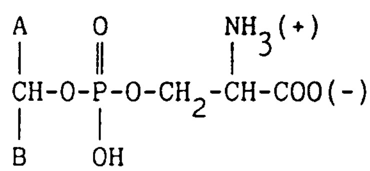

wherein A = H, $-(CH_2)_n$ -O -CO-$R_1$, n = 1 or 2,
and B = $-(CH_2)_m$ -O -CO-$R_2$, m = 1, 2, 3 or 4 with the limitation that, when A = $-(CH_2)_n$ - O-CO-$R_1$, m = 1 or 2, and wherein $R_1$ and $R_2$, equal or different, are radicals of acids of the aliphatic, aromatic, araliphatic, alicyclic, heteroyclic series and Z is H or OH, comprising reacting a phosphoric acid derivative of formula (II)

(II) 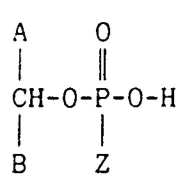

therein A and B have the defined meanings, with N-carbobenzyloxy-L-serine benzyl ester, in the presence of a condensing agent and catalytically hydrogenating the obtained compound (IV)

(IV) 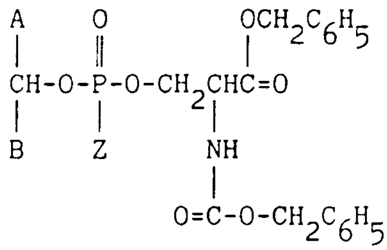

in which A and B have the previously defined meanings.

**2.** Process according to claim 1 characterized by:

a) reacting a phosphoric acid derivative of formula (II); wherein A,B,$R_1$ and $R_2$ have the above defined meanings, with N-carbobenzyloxy-L-serine-benzyl ester (III) in the presence of a condensing agent and of an inert organic solvent:

(II) 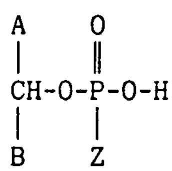 + 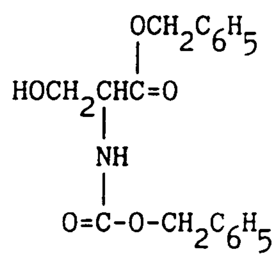 (III)

b) heating the obtained solution to evaporate the solvent

c) taking up the residue in a suitable organic solvent and purifying the solution, obtaining a compound of formula (IV)

(IV) 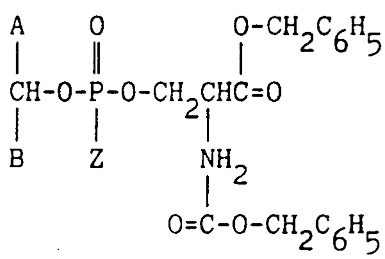

d) dissolving compound (IV) in a suitable organic solvent and hydrogenating it in the presence of a hydrogenation catalyst; when Z = H, phase d) is modified thus:

the purified fraction is dissolved in a suitable organic solvent and oxidized with a suitable oxidizing agent, the preferred oxidizing agent being iodine in watery pyridine.

e) purifying and concentrating the solution;

f) drying the obtained precipitate obtaining a compound of formula (V)

(V) 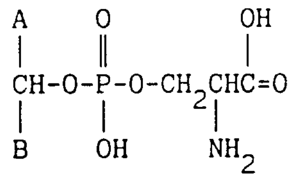

wherein A and B are as previously defined.

**3.** Process according to claim 2, characterized in that:
in stage a) suitable organic solvents may be selected from the group consisting of tertiary organic bases, preferably pyridine and quinoline;
in stage b) the heating is done first for a time of between approximately 10 and 60 minutes at a temperature between approximately 40° and approximately 80°C, under stirring and thereafter for a time of between 2 and 3 hours at room temperature;
in stage c) the residue is dried under reduced pressure, taken up in ethyl- or isopropyl ether, the precipitate is filtered off, the ether phase is washed with HCl and water and the solvent evaporated:
in stage d) the preferred organic solvent is acetic acid and the hydrogenation is performed with the aid of palladium on carbon;
in stage e) the solution is filtered on Celite (R) or on other filter aid and evaporated under reduced pressure.

**Patentansprüche**
**Patentansprüche für folgende Vertragsstaaten : AT, BE, CH, DE, DK, FR, GB, IT, LI, LU, NL, SE**

**1.** Serinderivate der allgemeinen Formel (I),

(I) 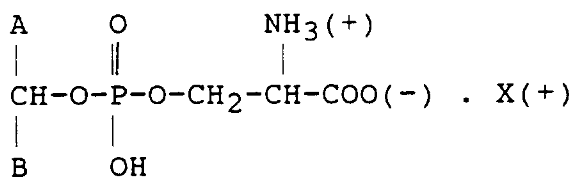

in der A H, $-(CH_2)_n-O-COR_1$, wobei n 1 oder 2 ist,
und B $-(CH_2)_m-O-CO-R_2$, wobei m 1, 2, 3 oder 4 ist, bedeuten, mit der Einschränkung, daß m 1 oder 2 ist, wenn A $-(CH_2)_n-O-COR_1$ bedeutet, und worin $R_1$ und $R_2$, die gleich oder verschieden sein können, Reste von Säuren der aliphatischen, aromatischen, alicyklischen und heterocyklischen Reihe bedeuten, und
X ein Alkali- oder Erdalkalimetall, ein primäres, sekundäres oder tertiäres Amin oder eine aliphatische, aromatische oder heterocyklische quaternäre Ammoniumbase darstellt.

**2.** Serinderivate gemäß Anspruch 1, dadurch gekennzeichnet, daß $R_1$ und $R_2$ Reste von aliphatischen gesättigten, einfach oder mehrfach ungesättigten Säuren der aliphatischen Reihe mit maximal 20 Kohlenstoffatomen sind.

**3.** Serinderivate gemäß Anspruch 1, dadurch gekennzeichnet, daß $R_1$ und $R_2$ Reste der Pivalinsäure, Laurinsäure, Myristinsäure, Palmitinsäure, Palmitoleinsäure, Stearinsäure, Ölsäure, Linolsäure, Linolensäure oder der Arachidonsäure sind.

**4.** Serinderivate gemäß Anspruch 1, dadurch gekennzeichnet, daß $R_1$ und $R_2$ Hydroxycarbonsäurereste sind.

**5.** Serinderivate gemäß Anspruch 1, dadurch gekennzeichnet, daß $R_1$ und $R_2$ Aminosäurereste sind.

**6.** Serinderivate gemäß Anspruch 1, dadurch gekennzeichnet, daß $R_1$ und $R_2$ Benzoesäurereste sind, die im Benzolring substituiert sein können.

**7.** Serinderivate gemäß Anspruch 1, dadurch gekennzeichnet, daß $R_1$ und $R_2$ Dicarbonsäurereste sind.

**8.** Verfahren zur Herstellung von Serinderivaten der allgemeinen Formel (I) ,

(I) 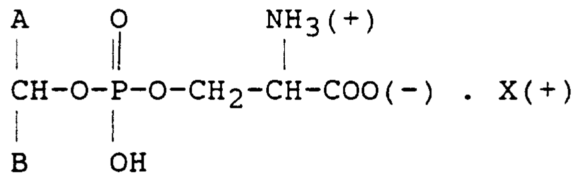

in der A H, $-(CH_2)_n$-O-$COR_1$, wobei n 1 oder 2 ist,
und B $-(CH_2)_m$-O-CO-$R_2$, wobei m 1, 2, 3 oder 4 ist, bedeuten, mit der Einschränkung, daß m 1 oder 2 ist, wenn A $-(CH_2)_n$-O-$COR_1$ bedeutet, und worin $R_1$ und $R_2$, die gleich oder verschieden sein können, Reste von Säuren der aliphatischen, aromatischen, alicyklischen und heterocyklischen Reihe bedeuten und Z H oder OH ist, welches die Umsetzung eines Phosphorsäure-derivats der Formel (II)

(II) 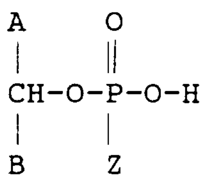

worin A und B die oben definierten Bedeutungen aufweist, mit N-Carbobenz-oxy-L-serinbenzylester in Gegenwart eines Kondensationsmittels und die katalytische Hydrierung der erhaltenen Verbindung (IV)

(IV) 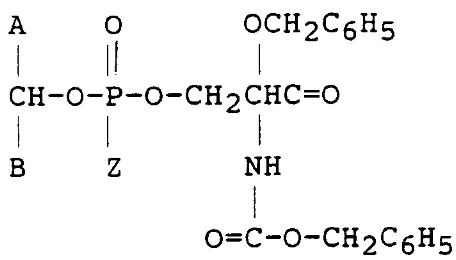

wobei A und B die oben definierten Bedeutungen aufweisen, umfaßt.

**9.** Verfahren gemäß Anspruch 8, dadurch gekennzeichnet, daß
a) ein Phosphorsäurederivat der Formel (II), in der A, B, $R_1$ und $R_2$ die oben definierten Bedeutungen aufweisen, mit N-Carbobenzoxy-L-serin-benzylester (III) in Gegenwart eines Kondensationsmittels

(II) 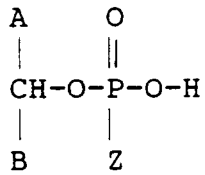 + 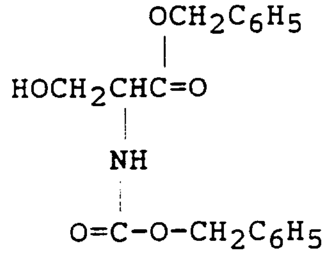 (III)

umgesetzt wird,

b) die erhaltene Lösung erhitzt wird, um das Lösungsmittel abzudampfen,

c) der Rückstand in einem geeigneten organischen Lösungsmittel aufgenommen und die Lösung gereinigt wird, wobei eine Verbindung der Formel (IV)

(IV) 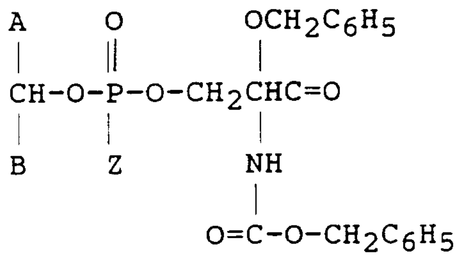

erhalten wird,

d) die Verbindung (IV) in einem geeigneten organischen Lösungsmittel gelöst und in Gegenwart eines Hydrierungskatalysators hydriert wird und die Stufe d) folgendermaßen modifiziert wird, falls Z H ist: die gereinigte Fraktion in einem geeigneten organischen Lösungsmittel gelöst und mit einem geeigneten Oxidationsmittel oxidiert wird, wobei das bevorzugte Oxidationsmittel Iod in wässrigem Pyridin ist,

e) die Lösung gereinigt und konzentriert wird,

f) der erhaltene Niederschlag getrocknet und eine Verbindung der Formel (V)

(V) 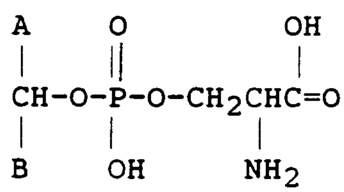

in der A und B die oben definierten Bedeutungen besitzen, erhalten wird.

**10.** Verfahren gemäß Anspruch 9, dadurch gekennzeichnet, daß
in der Stufe a) das geeignete organische Lösungsmittelaus der Gruppe ausgewählt werden kann, die aus tertiären organischen Basen, vorzugsweise Pyridin und Chinolin, besteht,
in der Stufe b) das Erwärmen zuerst unter Rühren für eine Zeit zwischen etwa 10 bis 60 Minuten bei einer Temperatur zwischen etwa 40°C und etwa 80°C und danach für eine Zeit zwischen 2 bis 3 Stunden bei Raumtemperatur durchgeführt wird,
in der Stufe c) der Rückstand unter vermindertem Druck getrocknet, in Ethyl- oder Isopropylether aufgenommen, der Niederschlag abfiltriert die Etherphase mit HCl gewaschen und das Wasser und das organische Lösungsmittel abgedampft wird,
in der Stufe d) das bevorzugte organische Lösungsmittel Essigsäure ist und die Hydrierung mittels Palladiumkohle durchgeführt wird,

in der Stufe e) die Lösung über Celit® oder ein anderes Filterhilfsmittel filtriert und unter vermindertem Druck eingedampft wird.

**11.** Pharmazeutische Zusammensetzungen, die als Wirkstoff zumindest eine Verbindung der allgemeinen Formel (I)

(I) 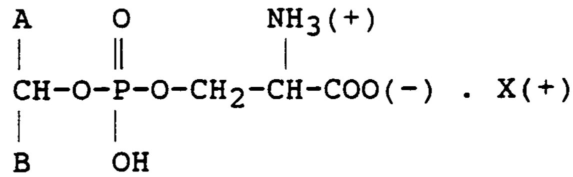

enthalten,
in der A H, $-(CH_2)_n-O-COR_1$, wobei n 1 oder 2 ist,
und B $-(CH_2)_m-O-CO-R_2$, wobei m 1, 2, 3 oder 4 ist, bedeuten, mit der Einschränkung, daß m 1 oder 2 ist, wenn A $-(CH_2)_n-O-COR_1$ bedeutet, und worin $R_1$ und $R_2$, die gleich oder verschieden sein können, Reste von Säuren der aliphatischen, aromatischen, alicyklischen und heterocyklischen Reihe bedeuten, und
X ein Alkali- oder Erdalkalimetall, ein primäres, sekundäres oder tertiäres Amin oder eine aliphatische, aromatische oder heterocyklische quaternäre Ammoniumbase darstellt,
die in der Therapie von humanen pathologischen Zuständen, welche von einer Verlangsamung des cerebralen Metabolismus begleitet sind, und als hypolipidämische Mittel wirksam sind.

**12.** Pharmazeutische Zusammensetzungen gemäß Anspruch 11, dadurch gekennzeichnet, daß $R_1$ und $R_2$ Reste von aliphatischen gesättigten, einfach oder mehrfach ungesättigten Säuren mit maximal 20 Kohlenstoffatomen und vorzugsweise Reste der Pivalinsäure, Laurinsäure, Myristinsäure, Palmitinsäure, Palmitoleinsäure, Stearinsäure, Ölsäure, Linolsäure, Linolensäure oder der Arachidonsäure sind.

**13.** Pharmazeutische Zusammensetzungen gemäß Anspruch 11, dadurch gekennzeichnet, daß $R_1$ und $R_2$ Reste von Hydroxycarbonsäuren, Aminosäuren. Dicarbonsäuren und von einfachen oder substituierten aromatischen Carbonsäuren sind.

**14.** Pharmazeutische Zusammensetzungen gemäß Anspruch 11, dadurch gekennzeichnet, daß sie gegen für das Alter typische humane pathologische Zustände, gegen Alzheimer Krankheit, Gedächtnisverlust und senile Demenz allgemein, gegen anoxische und ödematöse Zustände allgemein, gegen cerebrale Ermüdung, neuroendokrine und immunologische Veränderungen wirksam ist.

**15.** Pharmazeutische Zusammensetzungen gemäß Anspruch 11, dadurch gekennzeichnet, daß der Wirkstoffgehalt zwischen 20 und 1000 mg liegt.

**16.** Pharmazeutische Zusammensetzungen gemäß Anspruch 15, dadurch gekennzeichnet, daß der Wirkstoffgehalt zwischen 40 und 350 mg liegt.

**Patentansprüche für folgende Vertragsstaaten : ES, GR**

**1.** Verfahren zur Herstellung von Serinderivaten der allgemeinen Formel (I) ,

(I) 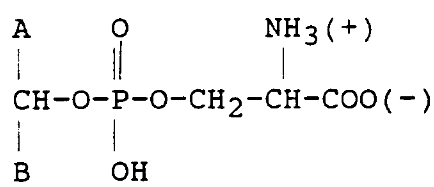

in der A H, $-(CH_2)_n-O-COR_1$, wobei n 1 oder 2 ist,

und B $-(CH_2)_m-O-CO-R_2$, wobei m 1, 2, 3 oder 4 ist, bedeuten, mit der Einschränkung, daß m 1 oder 2 ist, wenn A $-(CH_2)_n-O-COR_1$ bedeutet, und worin $R_1$ und $R_2$, die gleich oder verschieden sein können, Reste von Säuren der aliphatischen, aromatischen, alicyklischen und heterocyklischen Reihe bedeuten und Z H oder OH ist, welches die Umsetzung eines Phosphorsäure-derivats der Formel (II)

(II) 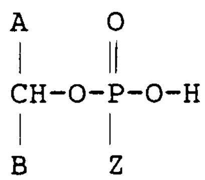

worin A und B die oben definierten Bedeutungen aufweist, mit N-Carbobenz-oxy-L-serinbenzylester in Gegenwart eines Kondensationsmittels und die katalytische Hydrierung der erhaltenen Verbindung (IV)

(IV) 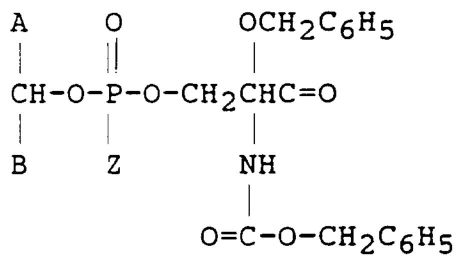

wobei A und B die oben definierten Bedeutungen aufweisen, umfaßt.

**2.** Verfahren gemäß Anspruch 1, dadurch gekennzeichnet, daß

a) ein Phosphorsäurederivat der Formel (II), in der A, B, $R_1$ und $R_2$ die oben definierten Bedeutungen aufweisen, mit N-Carbobenzoxy-L-serin-benzylester (III) in Gegenwart eines Kondensationsmittels

(II) 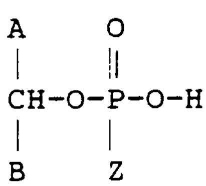 + 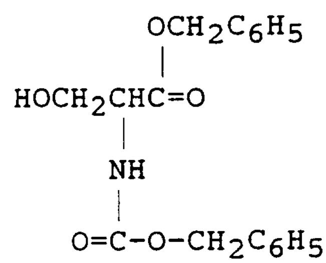 (III)

umgesetzt wird,

b) die erhaltene Lösung erhitzt wird, um das Lösungsmittel abzudampfen, c) der Rückstand in einem geeigneten organischen Lösungsmittel aufgenommen und die Lösung gereinigt wird, wobei eine Verbindung der Formel (IV)

(IV) 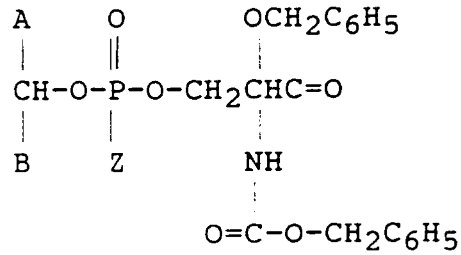

erhalten wird,
d) die Verbindung (IV) in einem geeigneten organischen Lösungsmittel gelöst und in Gegenwart eines Hydrierungskatalysators hydriert wird und die Stufe d) folgendermaßen modifiziert wird, falls Z H ist: die gereinigte Fraktion in einem geeigneten organischen Lösungsmittel gelöst und mit einem geeigneten oxidationsmittel oxidiert wird, wobei das bevorzugte oxidationsmittel Iod in wässrigem Pyridin ist,
e) die Lösung gereinigt und konzentriert wird,
f) der erhaltene Niederschlag getrocknet und eine Verbindung der Formel (V)

(V) 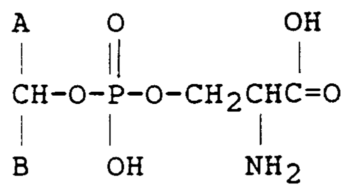

in der A und B die oben definierten Bedeutungen besitzen, erhalten wird.

**3.** Verfahren gemäß Anspruch 2, dadurch gekennzeichnet, daß
in der Stufe a) das geeignete organische Lösungsmittelaus der Gruppe ausgewählt werden kann, die aus tertiären organischen Basen, vorzugsweise Pyridin und Chinolin, besteht,
in der Stufe b) das Erwärmen zuerst unter Rühren für eine Zeit zwischen etwa 10 bis 60 Minuten bei einer Temperatur zwischen etwa 40 °C und etwa 80 °C und danach für eine Zeit zwischen 2 bis 3 Stunden bei Raumtemperatur durchgeführt wird,
in der Stufe c) der Rückstand unter vermindertem Druck getrocknet, in Ethyl- oder Isopropylether aufgenommen, der Niederschlag abfiltriert die Etherphase mit HCl gewaschen und das Wasser und das organische Lösungsmittel abgedampft wird,
in der Stufe d) das bevorzugte organische Lösungsmittel Essigsäure ist und die Hydrierung mittels Palladiumkohle durchgeführt wird,
in der Stufe e) die Lösung über Celit® oder ein anderes Filterhilfsmittel filtriert und unter vermindertem Druck eingedampft wird.

**Revendications**
**Revendications pour les Etats contractants suivants : AT, BE, CH, DE, DK, FR, GB, IT, LI, LU, NL, SE**

**1.** Dérivés de sérine de formule générale (I)

(I) 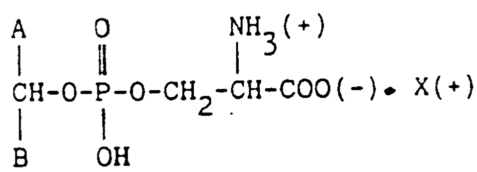

dans laquelle A = H, $-(CH_2)_n-O-CO-R_1$, n égal 1 ou 2,
et B = $-(CH_2)_m-O-CO-R_2$, m étant égal à 1, 2, 3 ou 4, sous réserve que, lorsque A = $-(CH_2)_n$ O-CO-$R_1$, m = 1 ou 2 et dans laquelle $R_1$ et $R_2$ identiques ou différents, sont des radicaux d'acides des

séries aliphatique, aromatique, araliphatique, alicyclique ou hétérocyclique, X étant un métal alcalin ou alcalino-terreux, une amine primaire, secondaire, tertiaire ou un ammonium quaternaire aliphatique, aromatique ou hétérocyclique.

**2.** Dérivés de sérine selon la revendication 1, caractérisés en ce que $R_1$ et $R_2$ sont des radicaux d'acides aliphatiques saturés mono- ou poly-insaturés des séries aliphatiques renfermant un maximum de 20 atomes de carbone.

**3.** Dérivés de sérine selon la revendication 1, caractérisés en ce que $R_1$ et $R_2$ sont des radicaux d'acides pivalique, laurique, myristique, palmitique, palmitoléique, stéarique, oléique, linoléique, linolénique ou arachidonique.

**4.** Dérivés de sérine selon la revendication 1, caractérisés en ce que $R_1$ et $R_2$ sont des radicaux hydroxyacides.

**5.** Dérivés de sérine selon la revendication 1, caractérisés en ce que $R_1$ et $R_2$ sont des radicaux aminoacides.

**6.** Dérivés de sérine selon la revendication 1, caractérisés en ce que $R_1$ et $R_2$ sont des radicaux d'acides benzoïques qui peuvent être substitués sur le cycle du benzène.

**7.** Dérivés de sérine selon la revendication 1, caractérisés en ce que $R_1$ et $R_2$ sont des radicaux de diacides.

**8.** Procédé de préparation de dérivés de sérine de formule générale (I)

(I) 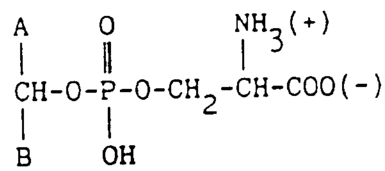

dans laquelle A = H, $-(CH_2)_n-O-CO-R_1$, n égal 1 ou 2,

et B = $-(CH_2)_m-O-CO-R_2$, m étant égal à 1, 2, 3 ou 4, sous réserve que, lorsque A = $-(CH_2)_n-O-CO-R_1$, m = 1 ou 2 et dans laquelle $R_1$ et $R_2$, identiques ou différents, sont des radicaux d'acides des séries aliphatique, aromatique, araliphatique, alicyclique ou hétérocyclique, et Z représente H ou OH, consistant à faire réagir un dérivé d'acide phosphorique de formule (II)

(II) 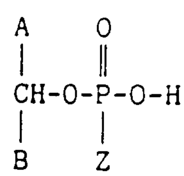

dans laquelle A et B ont les significations ci-dessus, avec du benzyl ester de N-carbobenzyloxy-L-sérine, en présence d'un agent de condensation et à hydrogéner catalytiquement le dérivé (IV) obtenu

(IV) 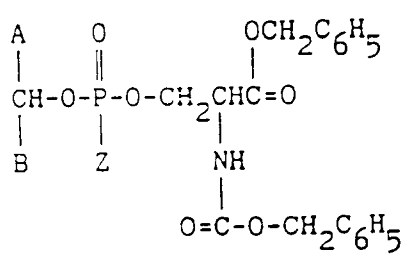

dans lequel A et B ont les significations définies ci-dessus.

**9.** Procédé selon la revendication 8, caractérisé en ce que:

a) on fait réagir un dérivé d'acide phosphorique de formule (II), dans laquelle A,B,$R_1$ et $R_2$ ont les significations définies ci-dessus, avec du benzyl ester de n-carbobenzyloxy-L-sérine (III) en présence d'un agent de condensation et d'un solvant organique inerte:

(II) 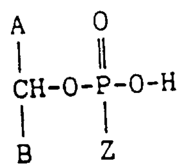 + 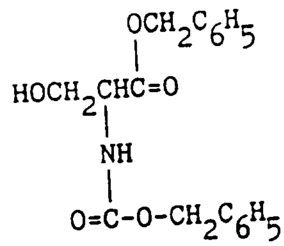 (III)

b) chauffage de la solution obtenue pour évaporer le solvant.

c) reprise du résidu dans un solvant organique et purification de la solution, pour obtenir un dérivé de formule (IV)

(IV) 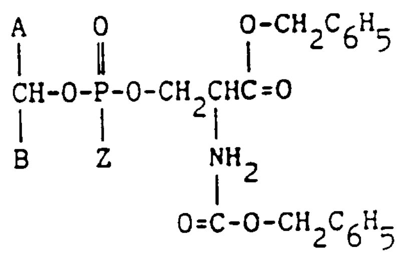

d) dissolution du composé (IV) dans un solvant organique convenable et hydrogénation en présence d'un catalyseur d'hydrogénation; lorsque Z = H, la phase d) est ainsi modifiée:

la fraction purifiée est dissoute dans un solvant organique convenable et oxydée par un agent d'oxydation convenable, l'agent d'oxydation préféré étant l'iode dans de la pyridine aqueuse.

e) purification et concentration de la solution;

f) séchage du précipité obtenu pour obtenir un dérivé de formule (V)

(V) 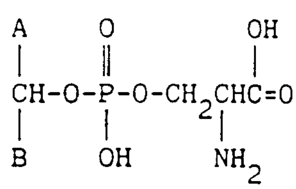

dans laquelle A et B sont tels que définis ci-dessus.

**10.** Procédé de fabrication selon la revendication 9, caractérisé en ce que:

dans l'étape a) les solvants organiques peuvent être choisis dans le groupe constitué par les bases organiques tertiaires, de préférence pyridine et quinoléine;

dans l'étape b) le chauffage est réalisé tout d'abord pendant un temps compris entre approximativement 10 et 60 minutes à une température comprise entre approximativement 40 et approximativement 80 ° C, sous agitation et, ensuite, pendant une durée comprise 2 et 3 heures, à la température ambiante;

dans l'étape c) le résidu est séché sous pression réduite, repris dans l'éthyl éther ou l'isopropyl éther, le précipité est éliminé par filtration, la phase éther est lavé par HCl et par l'eau et le solvant est évaporé;

dans l'étape d) le solvant organique préféré est l'acide acétique et l'hydrogénation est mise en oeuvre avec l'aide du palladium sur carbone;

dans l'étape e) la solution est filtrée sur Célite® ou sur un autre adjuvant de filtration et évaporée sous pression réduite.

**11.** Compositions pharmaceutiques contenant comme principe actif au moins un dérivé de formule générale (I)

(I) 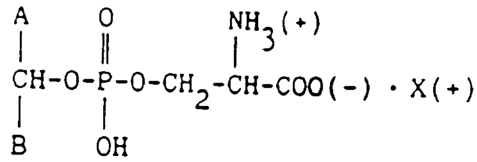

dans laquelle A = H, $-(CH_2)_n-O-CO-R_1$, n égal 1 ou 2,

et B = $-(CH_2)_m-O-CO-R_2$, m étant égal à 1, 2, 3 ou 4, sous réserve que, lorsque A = $-(CH_2)_n-O-CO-R_1$, m = 1 ou 2 et dans laquelle $R_1$ et $R_2$, identiques ou différents, sont des radicaux d'acides des séries aliphatique, aromatique, araliphatique, alicyclique ou hétérocyclique, X étant une un métal alcalin ou alcalino-terreux, une amine primaire, secondaire, tertiaire ou d'un ammonium quaternaire, aliphatique, aromatique ou hétérocyclique, active en thérapeutique de pathologies humaines associées à un ralentissement du métabolisme cérébral et comme agents d'hypolipidémisation.

**12.** Compositions pharmaceutiques selon la revendication 11, caractérisées en ce que $R_1$ et $R_2$ sont des radicaux des acides aliphatiques saturés, mono- ou poly-insaturés renfermant un maximum de 20 atomes de carbone, et de préférence des radicaux des acides pivaliques, laurique, myristique, palmitique, palmitoléique, stéarique, oléique, linoléique, linolénique ou arachidoniques.

**13.** Compositions pharmaceutiques selon la revendication 11, caractérisées en ce que $R_1$ et $R_2$ sont des radicaux des hydroacides, aminoacides, diacides, acides aromatiques simples ou substitués.

**14.** Compositions pharmaceutiques selon la revendication 11, caractérisées en ce qu'ils sont actifs contre les pathologies humaines typiques de l'âge avancé, maladie d'Alzheimer, perte de mémoire et démence sénile en général, situations anoxiques et oedématiques en général, fatigue cérébrale, désordres neuroendocrinien et immunologique.

**15.** Compositions pharmaceutiques selon la revendication 11, caractérisées en ce que les teneurs en principe actif sont comprises entre 20 et 1000 mg.

**16.** Compositions pharmaceutiques selon la revendication 15, caractérisées en ce que les teneurs en principe actif sont comprises entre 40 et 350 mg.

**Revendications pour les Etats contractants suivants : ES, GR**

**1.** Procédé de préparation de dérivés de sérine de formule générale (I)

(I) 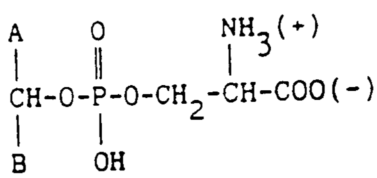

dans laquelle A = H, $-(CH_2)_n-O-CO-R_1$, n égal 1 ou 2,

et B = $-(CH_2)_m-O-CO-R_2$, m étant égal à 1, 2, 3 ou 4, sous réserve que, lorsque A = $-(CH_2)_n-O-CO-R_1$, m = 1 ou 2 et dans laquelle $R_1$ et $R_2$, identiques ou différents, sont des radicaux d'acides des séries aliphatique, aromatique, araliphatique, alicyclique ou hétérocyclique, et Z représente H ou OH, consistant à faire réagir un dérivé d'acide phosphorique de formule (II)

(II) 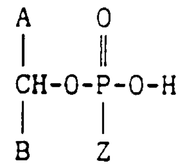

dans laquelle A et B ont les significations ci-dessus, avec du benzyl ester de N-carbobenzyloxy-L-sérine, en présence d'un agent de condensation et à hydrogéner catalytiquement le dérivé (IV) obtenu

(IV) 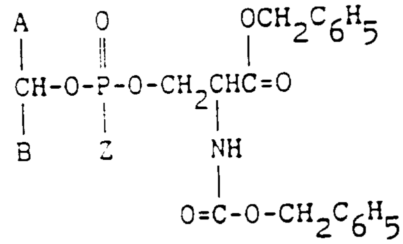

dans lequel A et B ont les significations définies ci-dessus.

**2.** Procédé selon la revendication 1, caractérisé en ce que:

a) on fait réagir un dérivé d'acide phosphorique de formule (II), dans laquelle A,B,$R_1$ et $R_2$ ont les significations définies ci-dessus, avec du benzyl ester de n-carbobenzyloxy-L-sérine (III) en présence d'un agent de condensation et d'un solvant organique inerte:

(II) 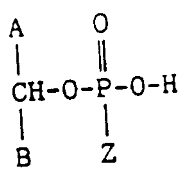 + 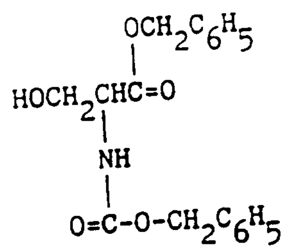 (III)

b) chauffage de la solution obtenue pour évaporer le solvant.

c) reprise du résidu dans un solvant organique et purification de la solution, pour obtenir un dérivé de formule (IV)

(IV) 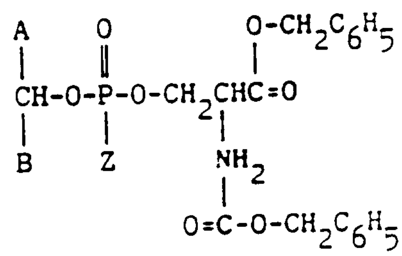

d) dissolution du composé (IV) dans un solvant organique convenable et hydrogénation en présence d'un catalyseur d'hydrogénation; lorsque Z = H, la phase d) est ainsi modifiée:

la fraction purifiée est dissoute dans un solvant organique convenable et oxydée par un agent d'oxydation convenable, l'agent d'oxydation préféré étant l'iode dans de la pyridine aqueuse.

e) purification et concentration de la solution;

f) séchage du précipité obtenu pour obtenir un dérivé de formule (V)

(V) 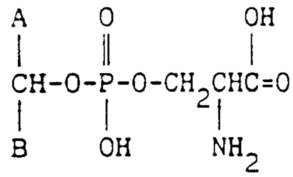

dans laquelle A et B sont tels que définis ci-dessus.

**3.** Procédé de fabrication selon la revendication 2, caractérisé en ce que:

dans l'étape a) les solvants organiques peuvent être choisis dans le groupe constitué par les bases organiques tertiaires, de préférence pyridine et quinoléine;

dans l'étape b) le chauffage est réalisé tout d'abord pendant un temps compris entre approximativement 10 et 60 minutes à une température comprise entre approximativement 40 et approximativement 80°C, sous agitation et, ensuite, pendant une durée comprise 2 et 3 heures, à la température ambiante;

dans l'étape c) le résidu est séché sous pression réduite, repris dans l'éthyl éther ou l'isopropyl éther, le précipité est éliminé par filtration, la phase éther est lavé par HCl et par l'eau et le solvant est évaporé;

dans l'étape d) le solvant organique préféré est l'acide acétique et l'hydrogénation est mise en oeuvre avec l'aide du palladium sur carbone;

dans l'étape e) la solution est filtrée sur Célite® ou sur un autre adjuvant de filtration et évaporée sous pression réduite.